# DEMANDE DE BREVET EUROPEEN

(11) **EP 0 588 738 A1**
(43) Date de publication de la demande: **23.03.1994**
(21) Numéro de dépôt: 93430010.4
(22) Date de dépôt: 22.07.1993
(51) Int. Cl.: C03C 13/04, A61B 17/36, H01S 3/16

(54) **Dispositifs optiques pour transmission laser**

(30) Priorité: 23.07.1992 US 917285
(71) Demandeur: LASER MEDICAL TECHNOLOGY, Inc., San Clemente, California 92672 (US)
(72) Inventeur: Levy, Guy, Tustin, California 92680 (US)
(74) Mandataire: Marek, Pierre

(57) **Abrégé**

Dispositif optique pour la transmission d'un rayonnement laser, comprenant un élément de transmission de lumière, caractérisé en ce que cet élément (4, 8) contient du fluorure de calcium. L'invention concerne également un appareil laser, principalement pour interventions médicales ou dentaires, comprenant un laser (2) et un dispositif optique (4, 8) pour conduire le rayonnement émis par ledit laser, jusqu'à la zone à traiter, réalisé de la manière indiquée ci-dessus.

## Description

La présente invention concerne un dispositif optique destiné à l'application d'un rayonnement laser, en particulier pour des traitements médicaux et dentaires, ce dispositif pouvant être avantageusement réalisé sous forme d'une fibre optique. L'invention concerne également les appareils ou installations laser comportant application de ce dispositif optique.

On sait que les fibres optiques fournissent un moyen efficace pour appliquer un rayonnement laser sur des régions à traiter dans le cadre d'opérations ou procédures médicales et dentaires. Ces opérations comprennent, entre autres, la coupe de tissus durs et mous, la cautérisation et la stérilisation. Les recherches concernant les effets du rayonnement laser ont abouti au développement d'opérations ou procédures qui permettent d'accroître les niveaux d'énergie d'un tel rayonnement, qu'il est possible d'utiliser avec sécurité et efficacité pour une variété d'interventions. On peut s'attendre à ce que les prochaines études conduisent à d'autres augmentations des quantités d'énergie utilisables et capables de produire des résultats utiles.

Toutefois, l'un des obstacles que rencontre ce progrès attendu, est constitué par les niveaux ou quantités d'énergie que les fibres optiques ne peuvent transmettre sans être endommagées ou détruites. Alors qu'il est connu que le niveau d'énergie de rayonnement qui peut être acheminé par une fibre optique est proportionnel au diamètre de celle-ci, de sorte que le niveau d'énergie qui peut être conduit par une fibre peut être relevé en augmentant le diamètre de cette fibre, des fibres de gros diamètre sont désavantageuses pour plusieurs des applications auxquelles on se réfère dans la suite du présent exposé. A titre d'exemple, les fibres utilisées dans le cas de traitements dentaires, en particulier des traitements endodontiques, doivent avoir de très petits diamètres. Il en va de même lorsque les fibres doivent être mises en oeuvre pour conduire un rayonnement laser à travers des vaisseaux sanguins ou d'autres passages corporels. De plus, la souplesse de ces fibres diminue à mesure que leur diamètre augmente.

En conséquence, des efforts continus ont été faits en vue de mettre à la disposition des praticiens des compositions de fibres optiques, capables de conduire de hauts niveaux d'énergie d'émission laser sans être endommagées.

L'invention a pour but essentiel de fournir des compositions de fibres optiques qui possèdent cette caractéristique.

L'invention a pour autre but de mettre à la disposition des praticiens, des nouvelles fibres optiques susceptibles d'être utilisées dans n'importe quel appareil de traitement médical ou dentaire pour conduire le rayonnement laser vers les régions du corps que l'on veut traiter.

L'invention a encore pour but de réaliser des fibres optiques susceptibles d'être utilisées avec pratiquement tous les types de sources laser.

Suivant l'invention, les buts ci-dessus et d'autres encore sont atteints grâce à une fibre optique de transmission de rayonnement laser, cette fibre comprenant un élément de transmission de la lumière qui contient du fluorure de calcium.

Les recherches opérées ont permis de découvrir que le fluorure de calcium est transparent à certaines longueurs d'onde de rayonnement laser et peut transmettre des niveaux élevés d'énergie sans subir de dommages.

En outre, le fluorure de calcium a un point de fusion plus bas que la silice et un niveau de compatibilité biologique élevé. Par conséquent, lorsque le rayonnement laser est appliqué, à partir d'une fibre constituée par ou contenant du fluorure de calcium, sur un tissu dentaire ou osseux, la chaleur générée par interaction du rayonnement avec ce tissu peut agir pour fondre l'extrémité distale, ou pointe, de la fibre, et la matière de la fibre fondue peut s'écouler dans un canal radiculaire ou dans une ouverture pratiquée dans la matière dentaire ou osseuse, pour fournir une obturation ou un revêtement dense, sans vide ou cavité et biologiquement compatible.

La figure 1 est une vue à caractère schématique d'un appareil de traitement médical ou dentaire qui utilise une fibre optique conforme à l'invention.

La figure 2 est une vue en coupe longitudinale d'un mode de réalisation d'une fibre conforme à l'invention.

La figure 3 est une vue en coupe longitudinale d'un deuxième mode de réalisation d'une fibre conforme à l'invention.

La figure 4 est une vue à caractère schématique d'un autre appareil médical équipé d'un mode de réalisation de l'invention.

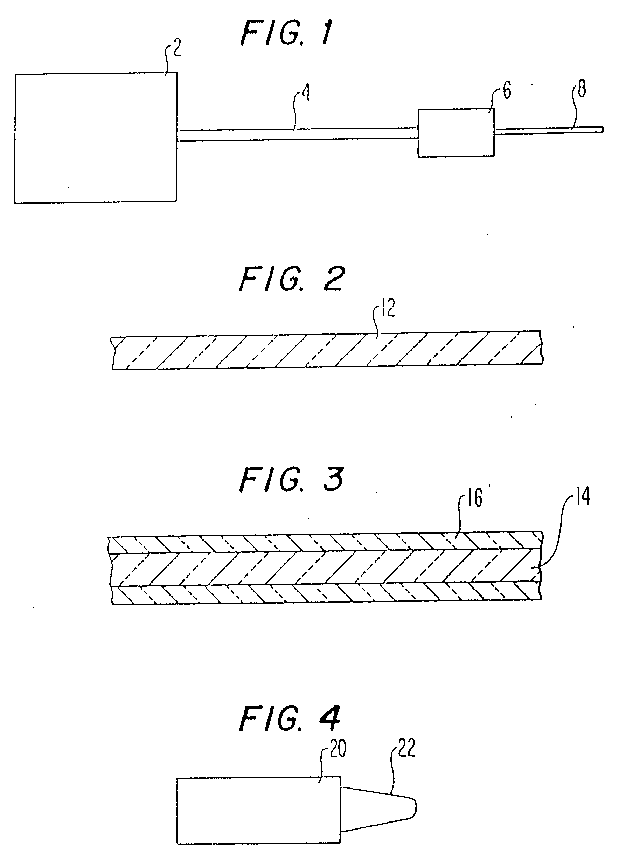

La figure 1 illustre, par une vue simplifiée, un appareil de traitement médical ou dentaire qui peut utiliser des fibres optiques conformes à l'invention. L'appareil comprend une unité laser 2, qui peut être constituée par n'importe quel type de laser actuellement utilisé ou étudié à des fins médicales ou dentaires, qui présente une sortie connectée à une fibre optique de transmission 4. La fibre 4 présente une extrémité de sortie montée dans une pièce à main 6, et une fibre de sortie 8 est couplée optiquement à l'intérieur de la pièce à main 6, à la fibre 4. La fibre 8 peut être utilisée directement en vue d'appliquer le rayonnement laser sur une région du corps qui doit être traitée, ou peut être introduite, par exemple par le biais d'un cathéter, dans un passage corporel, tel qu'un vaisseau sanguin, afin d'exécuter différents types de traitements. A titre d'alternative, le rayonnement peut être émis directement à partir de la pièce à main 6 ; dans ce cas, la fibre 8 est supprimée.

Selon une première disposition caractéristique de l'invention, la fibre 4 et/ou 8 est constituée par ou contient du fluorure de calcium cristallin, qui s'est avéré capable d'acheminer des niveaux de puissance de rayonnement laser extrêmement élevés, sans subir de dommage ou de destruction. Les recherches opérées démontrent que cet avantage est obtenu au moins pour la longueur d'onde YAG-Nd de 1,06µ , et l'on pense qu'un résultat similaire peut être atteint pour d'autres longueurs d'onde de rayonnement laser qui peuvent être transmises par l'intermédiaire de fibres conventionnelles à la silice.

Une fibre optique conforme à l'invention peut être une simple fibre 12 de section transversale homogène, comme illustrée par la figure 2, auquel cas elle peut être entièrement constituée de fluorure de calcium, avec des quantités d'impuretés à l'état de traces, ou contenir une proportion prédominante de fluorure de calcium, par exemple au moins 50 % de fluorure de calcium, ou contenir du fluorure de calcium comme ingrédient secondaire.

Il s'avère présentement que la capacité qu'ont les fibres selon l'invention à transmettre des niveaux d'énergie accrus dépend de leur teneur en fluorure de calcium ; on peut cependant apporter des améliorations appréciables à l'art antérieur même avec des fibres contenant moins de 50 % de fluorure de calcium.

Le mode de réalisation représenté sur la figure 3 se compose d'un noyau ou âme 14, enveloppée d'un revêtement ou gaine 16, l'une au moins de ces parties constitutives étant constituée par ou contenant du fluorure de calcium. Selon cette réalisation, l'âme 14 et le revêtement 16, ou l'un ou l'autre, peut avoir une composition telle que celle décrite précédemment, en relation avec la figure 2, les deux parties constituantes de la fibre ayant cependant des compositions différentes, en raison de la nature différente de la matière dans laquelle elle sont exécutées ou en raison de leur teneur différente en fluorure de calcium.

Selon les modes de réalisations préférés de l'invention, la fibre homogène de la figure 2, ou l'âme 14 et le revêtement ou gaine 16 de la figure 3, auront chacun la teneur maximale en fluorure de calcium, compatible avec d'autres propriétés optiques et physiques, y compris l'indice de réfraction, la flexibilité et la résistance à la tension.

En outre, une composition conforme à l'invention peut être utilisée comme pointe ou extrémité de contact pour une pièce à main utilisée en chirurgie au laser. La figure 4 illustre un tel dispositif, qui comprend une piéce à main 20 portant une pointe de contact 22. la pointe de contact 22, qui peut être jetable, a une composition telle que décrite ci-dessus, y compris une teneur en fluorure de calcium suffisante pour permettre à la pointe de conduire de hauts niveaux d'énergie de rayonnement laser sans subir de dommage ou de destruction. Une telle pointe constitue une alternative plus économique aux pointes de saphir actuellement utilisées dans les appareils de ce genre.

Lorsque la fibre 4 de la figure 1 a la forme montrée sur la figure 2, elle peut être constituée de fluorure de calcium, uniquement, ou d'un mélange de fluorure de calcium, et d'autres matériaux transparents aux rayonnements laser, comme la silice. Lorsque la fibre 4 d'acheminement du rayonnement laser de la figure 1 a la forme montrée sur la figure 3, l'âme 14 peut être constituée entièrement ou principalement de fluorure de calcium, et le revêtement 16 peut être constitué de silice ou d'autres matériaux ayant une bonne résistante mécanique.

La fibre optique 8 de la figure 1 peut avoir une extrémité distale conique, en particulier lorsqu'elle est utilisée pour élargir un canal radiculaire. Lorsque la fibre 8 d'application du rayonnement laser a la forme montrée sur la figure 2, elle peut être constituée entièrement de fluorure de calcium, ou d'un mélange de fluorure de calcium et d'autres matériaux, comme de la silice. Lorsque la fibre doit être utilisée pour obturer un canal radiculaire ou pour remplir ou recouvrir une cavité dans une dent ou dans un os, son extrémité distale, au moins, est, de préférence, constituée entièrement de fluorure de calcium. Lorsque la fibre 8 a la forme montrée sur la figure 3, au moins le revêtement 16 est entièrement constitué de fluorure de calcium. Le noyau ou âme 14 peut également être constitué de fluorure de calcium, ou d'un mélange de fluorure de calcium et d'autres matériaux ayant une bonne résistance mécanique, comme la silice, ou entièrement de ces autres matériaux.

En ce qui concerne la pointe de contact 22 illustrée sur la figure 4, elle peut être conique, comme représenté, ou non-conique, et peut être entièrement constituée de fluorure de calcium ou consister en un noyau d'autres matériaux, comme la silice, avec un revêtement de fluorure de calcium. Dans un cas comme dans l'autre, la chaleur générée lorsque le rayonnement laser émis depuis la pointe de contact réagit avec du tissu dentaire ou osseux peut faire fondre le fluorure de calcium, de manière à ce qu'il obture ou recouvre des cavités présentes dans ledit tissu.

Pour préparer du fluorure de calcium cristallin, une solution contenant des ions de calcium est mise à réagir avec une solution contenant des ions de fluorure. A titre d'exemple, l'addition d'une solution de nitrate de calcium à une solution de fluorure d'ammonium soluble aura pour effet de produire du fluorure de calcium, précipité sous forme de fluorure de calcium solide, et de NH₄NO₃.

Le fluorure de calcium solide précipité a la forme d'une poudre très fine. Pour produire de plus grosses granules, ou un corps solide de fluorure de calcium, on peut utiliser des techniques céramiques. Par exemple, une masse de fine poudre de fluorure de calcium peut être compressée pour former un gâteau de tourteau de couleur verte. Le tourteau de couleur verte peut ensuite être concrété pour former une pièce profilée de fluorure de calcium. Le point de fusion du fluorure de calcium reste proche de 1400 degrés C.

Après qu'une pièce profilée de fluorure de calcium a été produite de la manière décrite ci-dessus, elle peut être chauffée jusqu'à une température proche de son point de fusion, dans le but de ramollir le fluorure de calcium, sans le faire fondre. Pendant qu'elle est à l'état ramolli, la pièce profilée est étirée pour prendre approximativement la forme d'une fibre optique. La pièce profilée peut être extrudée autour d'un noyau ou âme de silice pour former une fibre représentée sur la figure 3. Puis, après refroidissement, la pièce est rectifiée et polie aux dimensions finales voulues.

A la fin du processus de fabrication précédemment décrit, la fibre de fluorure de calcium obtenue peut être enduite ou revêtue de plastique dur, de silice ou d'un autre matériau approprié. Ce revêtement, tel qu'il est représenté sur la figure 3, maintient l'intégrité structurelle d'une fibre de fluorure de calcium, pour permettre à cette fibre d'être pliée au moins dans une certaine mesure, en évitant la cassure ou l'endommagement du noyau ou âme de fluorure de calcium. Une fibre constituée uniquement par du fluorure de calcium a tendance à se rompre lorsqu'elle est pliée. Le revêtement de plastique dur, de silice ou d'un matériau similaire, tel qu'il est représenté sur la figure 3, tend à maintenir l'intégrité structurelle du noyau ou âme de fluorure de calcium.

Selon d'autres modes de réalisation de l'invention, la poudre de fluorure de calcium peut être mélangée à d'autres ingrédients avant d'être solidifiée ou concrétée. Ces autres ingrédiens peuvent être constitués par des matériaux choisis pour avoir la transparence requise au rayonnement laser à transmettre, silice par exemple, et capables de subir la concrétion et les opérations d'étirage ultérieures sans être endommagés ou détruits.

Bien que la description qui précède fasse référence à des réalisations spécifiques de la présente invention, il est entendu que de nombreuses modifications peuvent être apportées sans s'écarter de l'esprit de l'invention. Les revendications annexées sont destinées à couvrir toute modification de ce genre, qui serait comprise dans la portée et l'esprit conformes à l'invention.

Les réalisations dévoilées dans le présent brevet doivent donc être considérées à tous égards comme illustratives, et non limitatives, la portée de l'invention étant définie par les revendications annexées, plutôt que par la description qui précède, et toutes les modifications comprises dans l'esprit et dans l'étendue d'équivalence des revendications sont donc réputées comprises dans ces dernières.

## Revendications

**1. -** Dispositif optique pour la transmission d'un rayonnement laser, comprenant un élément de transmission de lumière, caractérisé en ce que cet élément (4, 8) contient du fluorure de calcium.

**2. -** Dispositif optique selon la revendication 1, caractérisé en ce que l'élément de transmission de Lumière est une fibre optique (4, 8).

**3. -** Dispositif optique suivant l'une des revendications 1 ou 2, caractérisé en ce qu'il comporte une âme ou noyau (14) et une couche de revêtement ou gaine (16) entourant ledit noyau (14), et en ce que l'une au moins de ces parties constitutives contient du fluorure de calcium.

**4. -** Dispositif optique selon la revendication 3, caractérisé en ce que seul le noyau ou âme (14) contient du fluorure de calcium.

**5. -** Dispositif optique suivant la revendication 3, caractérisé en ce que seul le revêtement ou gaine (16) contient du fluorure de calcium.

**6. -** Dispositif optique suivant la revendication 3, caractérisé en ce que le noyau ou âme (14) et le revêtement ou gaine (16) contiennent tous deux du fluorure de calcium.

**7. -** Dispositif optique selon l'une quelconque des revendications 1 à 6, caractérisé en ce que l'élément de transmission de lumière (4, 8), ou l'une et/ou l'autre des parties constitutives (14, 16) dudit élément de transmission de lumière (4, 8) contien(nen)t au moins 50 % en poids de fluorure de calcium.

**8. -** Dispositif optique selon l'une quelconque des revendications 1 à 7, caractérisé en ce que l'élément de transmission de lumière (4, 8) ou l'une au moins des parties constitutives (14, 16) dudit élément de transmission de lumière (4, 8) est constitué par un mélange de fluorure de calcium et d'un matériau transparent au rayonnement laser à transmettre.

**9. -** Dispositif optique selon l'une quelconque des revendications 1 à 5, caractérisé en ce que l'élément de transmission de lumière (4, 8) ou l'une au moins des parties constitutives (14, 16) dudit élément de transmission de lumière (4, 8), est constitué(e) entièrement ou quasi entièrement de fluorure de calcium.

**10. -** Appareil laser, principalement pour interventions médicales ou dentaires, comprenant un laser (2) et un dispositif optique (4, 8) pour conduire le rayonnement émis par ledit laser, jusqu'à la zone à traiter, caractérisé en ce que ledit dispositif optique est réalisé selon l'une quelconque des revendications 1 à 9.

**11. -** Appareil laser selon la revendication 10, caractérisé en ce que ledit dispositif optique (8) est constitué par une fibre optique dimensionnée pour pouvoir être introduite dans une cavité ou dans un passage anatomique.

**12. -** Appareil laser suivant la revendication 10, caractérisé en ce que ledit dispositif optique (8) constitue une pointe de contact (22) permettant d'appliquer le rayonnement laser sur la surface corporelle à traiter.
